# EUROPEAN PATENT SPECIFICATION

(11) **EP 1 758 637 B1**
(45) Date of publication and mention of the grant of the patent: **01.02.2012**
(21) Application number: 05755604.5
(22) Date of filing: 20.06.2005
(51) Int. Cl.: A61M 27/00, A61F 13/00

(54) **WOUND DRESSINGS FOR VACUUM THERAPY**
WUNDVERBÄNDE FÜR DIE VAKUUMTHERAPIE
PANSEMENT POUR THERAPIE PAR LE VIDE

(30) Priority: 21.06.2004 GB 0413867
(43) Date of publication of application: 07.03.2007
(73) Proprietor: Systagenix Wound Management IP Co. BV., 1077 XX Amsterdam (NL)
(72) Inventor: WATT, Paul, William, West Yorkshire BD20 6UN (GB); GREGORY, Sara, Jayne, West Yorkshire LS20 9EF (GB); TROTTER, Patrick, John, Leeds LS16 8BN (GB); SILCOCK, Derek, Walter, Skipton BD23 1QP (GB); MARSDEN, Donald, Christopher, Cononley BD20 8NY (GB)
(74) Representative: James, Anthony Christopher W.P.
(86) International application number: PCT/GB2005/002423
(87) International publication number: WO 2005/123170

(56) References cited:
- EP-B- 0 688 189
- WO-A-01/89431
- WO-A-02/083046
- WO-A-03/057307
- WO-A-2004/037334
- US-A- 4 382 441
- US-A- 5 645 081

## Description

The present invention relates to improved wound dressings for use in vacuum therapy of wounds. The invention also relates to wound treatment systems incorporating such dressings, and to kits for the manufacture of such dressings.

EP-A-062072-0 and EP-A-0688189 describe vacuum treatment for accelerating wound healing. They describe the use of a cover for sealing about the outer perimeter of the wound, under which a vacuum is established to act on the wound surface. This vacuum applied to the wound surface accelerates healing of chronic wounds. A screen of open cell foam material is provided under the cover to provide the space in which the vacuum is formed and to reduce tissue ingrowth. Sufficient vacuum is applied for a suitable duration to promote tissue migration in order to facilitate the closure of the wound. Suitable vacuum is between about 0.1 and 0.99 atmospheres. The vacuum can be substantially continuous, wherein the pressure is relieved only to change the dressing on the wound. Alternatively, the patent teaches cyclic application of vacuum in alternating periods of application and nonapplication. In a preferred embodiment, vacuum is applied in 5 minute periods of application and non-application.

WO01/89431 describes vacuum wound dressings further comprising a layer of a collagen scaffold material to promote wound healing. The preferred collagen material is small intestine submucosa (SIS).

WO2004/03733 describes an apparatus for cleansing wounds in which irrigant fluid from a reservoir is supplied to a conformable wound dressing, and wound exudate from the dressing is recirculated through a flow path which passes through the dressing. The apparatus further comprises a means, located outside the dressing, for cleansing the wound fluid before it is recirculated back to the dressing. The cleansing means removes materials deleterious to wound healing. It may comprise one or more of a microfiltration system, adsorption means and/or dialysis means. The cleansed fluid, still containing materials that are beneficial in promoting wound healing, is returned to the wound bed. The dressing may be a vacuum treatment dressing. The described apparatus is unsatisfactory in a number of respects. In particular, the wound is continuously immersed in the recycled liquid, which can result in maceration of skin around the wound an other problems. Furthermore, the cleansing equipment is complex, hard to clean, and requires a large volume of liquid to be recirculated.

WO 02/083046 describes a wound dressing system that includes a dressing and an irrigation system. The dressing includes a wound-contacting layer and an outer layer. The irrigation system is interposed between these two layers.

WO 03/057307 describes a wound vacuum therapy dressing kit for use with a wound drainage system. The kit includes a wound dressing member and various components or accessory items that are used in conjunction with the wound dressing member.

The present invention provides a wound dressing for vacuum therapy comprising: a cover configured for placement over a wound to maintain a reduced pressure over the wound and adapted for communication with a source of vacuum, and a screen structure for placement between the cover and the wound, characterized in that said screen structure comprises a freeze-dried sponge made by freeze-drying or solvent drying a dispersion of oxidized regenerated cellulose (ORC) with collagen or chitosan in water, whereby the screen structure is adapted to remove or inactivate undesirable components from the wound environment and/or to concentrate desirable components present in the wound environment.

The cover may be any one of the cover types described in the aforementioned patent applications EP-A-0620720, EP-A-0688189, WO01/89431, and WO2004/037334 relaxing to vacuum wound treatment . Briefly, the cover should be formed from substantially gas-impermeable material in order to be able to maintain a reduced pressure in the space over the wound being treated. Thermoplastic sheet materials of various types are suitable. The cover may suitably be substantially convex, and/or it may suitably be made of a semi-rigid material in order to help support the vacuum without collapsing. The cover may be provided with a layer of a medically acceptable pressure-sensitive adhesive on at least the periphery thereof for attachment of the cover to the skin around the wound to be treated. In other embodiments, the adhesive may be omitted and the cover sheet may be attached to the wound by suction.

Suitably, the wound dressing according to the present invention further comprises tubing for connecting the cover to the vacuum source. The connection is usually made through an aperture in the cover. The tubing may extend only outwardly of the cover, or it may extend inwardly through the cover into the vacuum wound treatment space. The dressing may be provided with a push, screw, snap or bayonet-type fitting for attachment of the vacuum tubing. The tubing may be connected to a fluid collection manifold located inside the cover sheet. The term "fluid collection manifold" refers to a hollow body having a plurality of apertures for collecting fluid from a plurality of locations under the cover sheet. The manifold may for example comprise an apertured envelope or a perforated spiral-wound tube. Other suitable fluid collection manifolds are described in WO2004/037334. The tubing may further be provided with a valve for controlling the application of vacuum. In certain embodiments the valve may be closed to maintain a desired atmosphere or pressure in the wound treatment space, or it may be a one-way or non-return valve to maintain reduced pressure over the wound after removal of the vacuum, source. The tubing and/or the cover sheet may be provided with a suitable coupling for attachment of a vacuum source.

The wound dressing of the present invention makes use of a screen structure that is adapted to removed or inactivate materials deleterious to wound healing and/or retain or concentrated materials that are beneficial in promoting wound healing. This screen structure therefore achieves not only the mechanical functions of supporting the cover sheet and filling the wound, but also enhances the metabolism wound healing without recourse to the complex external purification apparatus described in WO2004/037334.

It will be appreciated that the wound dressing according to the present invention may comprise, in addition to the oxidized regenerated cellulose (ORC), any of the screen components previously described for vacuum wound treatment in EP-A-0620720, BP-A4688189, WO01/89431, and WO2004/037334. Furthermore, the screen structure used in the present invention may be made by chemical modification or addition to any of the screens described in EP-A-0620720, EP-A-0688189, WO01/89431, and WO2004/037334. Suitable conventional screen components include foams formed of a polymeric material, such as polyurethane or polyester. Alternatively or additionally, the conventional portion of the screen may be in the form of, or comprise one or more conformable hollow bodies defined by a film, sheet or membrane, such as a bag, chamber, pouch or other structure, filled with a fluid or solid that urges it to the wound shape.

In mammals, injury triggers an organised complex cascade of cellular and biochemical events that result in a healed wound. Wound is a complex, dynamic process that results in the restoration of anatomic continuity and function; an ideally healed wound is one that has returned to normal anatomic structure, function and appearance. The wound fluid accordingly contains a complex and changing mixture of active components. Certain components are harmful to wound healing when present in excessive amounts. Other components of the wound fluid are known to promote wound healing.

For example, wound infection in acute and chronic wound is associated with elevated levels of protease enzymes, in particular of elastase. Chronic wounds, such as venous ulcers, pressure sores and diabetic ulcers, have a disordered wound healing metabolism even in the absence of infection. In particular, wound chronicity is associated with elevated levels of protease enzymes in the wound that interfere with the normal processes of tissue formation and destruction in the wound. The protease enzymes include collagenases and gelatinases, in particular matrix metalloproteinases 2 and 9 and elastase.

Accordingly, the screen structure in the wound dressings according to the present invention is adapted to remove or inactivate at least one endogenous protease enzyme present in wound fluid. In particular, it is adapted to remove or inactivate at least one endogenous protease enzyme selected from the group consisting of collagenases, gelatinases and elastases. The screen structure may remove or inactivate the protease enzymes, for example, by binding the enzymes to a solid substrate in the screen structure by ion exchange or affinity binding.

Concentrations of reactive oxygen species such as hydroxyl radicals (OH), singlet oxygen (¹O₂), hydroperoxyl radicals (-OOH), superoxideradical anions (-O₂⁻), and hydrogen peroxide (H₂O₂) can rise in damaged tissues, producing a condition known as oxidative stress. The presence of a low level of reactive oxygen species may be advantageous in the early stages of wound healing by both attracting and activating macrophages which engulf and kill bacteria and release cytokines and growth factors. Under mild oxidative stress conditions when hydrogen peroxide levels are slightly raised (around 10⁻⁸ to 10⁻⁴ molar), it has also been found that the rate of cell proliferation in fibroblast cultures is stimulated. However, prolonged and more severe oxidative stress may delay healing because it will produce chronic inflammation, divert available energy supply towards antioxidant defence at the expense of tissue reconstruction, and increase levels of matrix metalloproteinases which cause tissue breakdown. In more severe cases, elevated levels of reactive oxygen species can give rise to hydrogen peroxide-induced senescence or apoptosis (that is, programmed cell death) or tissue necrosis (that is, uncontrolled cell death and therefore permanent tissue damage).

Accordingly, the screen structure is adapted to remove or inactivate at least one oxidative free radical present in wound fluid. For example, the screen structure may comprise an antioxidant or free radical scavenger such as Vitamin C (ascorbic acid), retinoids such as Vitamin A, Vitamin E, hydroquinones, benzimidazoles, antioxidant-grafted polysaccharides such as those described in -US-A-5612321, aniline or acridine dyes, or mixtures or combinations thereof, along with ORC (which has been shown to have antioxidant properties).

A number of components of wound fluid are known, to promote wound healing, in particular the so-called growth factors. Accordingly, the screen structure is preferably adapted to increase the concentration of at least one growth factor in the wound fluid. In particular, it is preferably adapted to selectively bind at least one growth factor selected from the group consisting of platelet derived growth factor (PDGF), fibroblast growth factors (FGF), transforming growth factor beta (TGF-β), epidermal growth factor (EGF), vascular endothelial growth factor (VEGF) and insulin-like growth factor (IGF), and mixtures thereof , and allow their subsequent delivery back to the wound. For example, the screen may comprise a solid substrate to which the growth factors are bound by ion exchange, size exclusion, or affinity binding. The substrate may be biodegradable in the wound, thereby gradually releasing the growth factors back into the wound. In other embodiments the growth factors may be released back into the wound by addition of a dissociation buffer.

The screen structure is adapted to remove water from the wound fluid, thereby further increasing the concentration of wound healing factors at the wound surface. For example the screen structure may comprise a molecular sieve drying agent, or a hydrogel drying agents

In certain embodiments the screen structure may be adapted to filter the wound fluid to remove solid particles, debris, cells and even microorganisms from the wound fluid. Advantageously, the screen structure may be adapted to remove or inactivate at least one infective microorganism. The screen structure may be adapted to remove or inactivate at least one bacterial endotoxin, for example The screen structure may comprise a peptidomimetic or a positively charged material that binds to the negatively charged lipopolysaccharide endotoxins.

It is recognised that availability of iron is essential for the survival, replication, and differentiation of invading micro-organisms, Many micro-organisms can either secrete their own siderophores or utilise the siderophores secreted by other micro-organisms For the purpose of scavenging iron from their surroundings. It therefore appears that removal of iron (which may be present as free Fe²⁺/F³⁺ ions or in weak association with a complexant) from damaged tissue could assist in the prevention and treatment of infection by micro-organisms such as bacteria and yeasts.

Accordingly, the screen structure may be adapted to remove or inactivate at least one dissolved iron species in the wound fluid. Preferably, the iron is removed preferentially over other multivalent ions, such as zinc, that are beneficial to wound healing. Suitable iron sequestering substances include iron chelators such as desferrioxamine, and oxidized regenerated cellulose, which has been shown to sequester iron selectively over Zinc. Resorbable wound dressing materials comprise, or consist essentially of oxidized regenerated cellulose (ORC). ORC is usually made by oxidation of a regenerated cellulose fabric or fibers with dinitrogen tetroxide. It is available as loose fibers, fabrics (such as the fabrics available under the Registered Trade Marks SURGICEL, NU-KNIT and INTERCEED from Johnson & Johnson), or sponges. Advantages of fibrillar ORC include that it is resorbable and the degradation products stimulate cell proliferation and chemotaxis. ORC is an effective haemostat and is bactericidal. ORC can modify the pH of the wound environment, which in turn may stimulate chronic wound repair.

Data presented in EP-A-0919548 have shown that ORC alone can positively influence the wound environment by modulating growth factor function and inactivating proteases. The data also show that ORC binds to growth factors present in wound fluid. The gradual breakdown of the ORC in vivo then releases the growth factors back into the wound, thereby promoting wound heating.

Wound dressings of the present invention are complexes of ORC with collagen or chitosan, in particular freeze-dried sponges made by freeze-drying or solvent-drying dispersions of ORC with collagen or chitosan in a suitable solvent such as water. Complexes of this type are described in detail in W098/00180 .Freeze-dried collagen/ORC sponges are commercially available from Johnson & Johnson Medical Limited under the Registered Trade Mark PROMOGRAN, and are described in detail in EP-A-091 8548 and EP-A-1153622. The sponges can be modified to ensure they last longer at the wound site by changes in the manufacturing process eg increased chemical cross-linking, increase in solids content and/or increase in thickness of the final product. The biocompatible sponge screen is soft, conformable and biocompatible. It encourages cellular ingrowth and repair. The bioresorbable sponges can be left in the wound after removal of the vacuum or can slowly disappear during use. The sponge provides a filter to ensure that growth factors are retained at the wound site, while allowing for efficient removal of wound exudate effectively concentrating the growth factors which would otherwise be removed from the wound.

In some embodiments, the screen structure may comprise a molecular sieve material that is adapted to absorb water but not higher molecular weight components of the wound fluid, such as growth factors. In other embodiments, the screen structure in the dressings according to the present invention comprises a gel-forming polymer (Hydrogel) that absorbs water from the wound fluid to form a gel.

The term "gel-forming polymer" refers to medically acceptable macromolecular substances that form a gel with water under physiological conditions of temperature and pH. Such hydrogels preferably have the ability to swell and absorb fluid while maintaining a strong integral structure. Preferably, the hydrogel composition forms a gel that is substantially insoluble in water under physiological conditions, whereby the hydrogel is not washed away by the wound fluid.

Exemplary insoluble gels include certain cross-linked polyacrylate gels, calcium alginate gels, cross-linked hyaluronate gels, wherein the hydrogel layer comprises a hydrogel material selected from gels formed from vinyl alcohols, vinyl esters, vinyl ethers and carboxy vinyl monomers, meth(acrylic) acid, acrylamide, N-vinyl pyrrolidone, acylamidopropane sulphonic acid, PLURONIC (Registered Trade Mark) (block polyethylene glycol, block polypropylene glycol) polystyrene-, maleic acid, NN-dimothylacrylamide diacetone acrylamide, acryloyl morpholine, and mixtures thereof.

Suitably, the hydrogel comprises a hydrogel material selected from polyurethane gels, biopolymer gels, carboxymethyl cellulose gels, hydroxyethyl cellulose gels, hydroxy propyl ethyl cellulose, modified acrylamide and mixtures thereof. Suitable biopolymer gels include alginates, pectins, galactomannans, chitosan, gelatin, hyaluronates and mixtures thereof. Some of these biopolymer materials also promote wound heating. In certain embodiments, the hydrogel comprises a hydrogel material of the kind described in WO00/07638.In other embodiments, the gel-forming polymer may comprise or consist essentially of a superabsorbent polymer.

The gels may be cross-linked, and the cross-linking may be either covalent or ionic. The hydrogel material may further comprise from 5 to 50% by weight on a dry weight basis of one or more humectants such as glycerol. The hydrogel material may be supported or reinforced by a support layer such as textile filaments. In certain embodiments, the gel-forming fibers such as superabsorbent fibers may be formed into a woven or nonwoven fabic, optionally in admixture with textile fibers to reinforce the superabsorbent.

The use of the gel-forming polymer retains the wound fluid within dressing, so that no collection chamber is needed in the vacuum line, and fewer components are needed for the device. The vacuum tubing and pump remain uncontaminated by wound fluid. A smaller pump can be used, giving greater mobility for patient. A static vacuum can be applied and then the pump disconnected leaving negative pressure still maintained within the dressing,

In especially suitable embodiments, the screen structure comprises, or consists essentially of, a solid hydrogel layer having fluid flow passages extending therethrough. The gel screen may comprise a network of channels or tubes within the screen for the passage of fluid, thereby preventing gel blocking. In certain embodiments the channels may be formed by selectively cross-linking the gel. The channels can for example be formed by piercing a gel slab with a metallic rod or wire and then applying a voltage to the wire. This will result in a rapid increase in temperature of the rod or wire thus effectively forming a tube which is more resilient than the surrounding gel/bioabsorbable. By piercing the gel/bioabsorbable multiple times and in various directions and then cross-linking, a resilient tubular network will be formed. This will have the advantages of: supporting the gel/bioabsorbable so that it is not removed by the vacuum device; allowing more efficient passage of fluid through the gel, thus allowing a lower vacuum to be used; allowing a bioabsorbable gel to be used if required as the tubular network will stay in place, thus utilising any potential advantages of the bioabsorbable material; and extending the lifetime of the gel.

Any of the component materials making up the screen structure may comprise an antimicrobial material. Suitable antimicrobial agents may be selected from the group consisting of antiseptics and antibiotics and mixtures thereof. Suitable antibiotics include peptide antimicrobials (e.g. defensins, Magainin, synthetic derivatives of them) tetracycline, penicillins, terramycins, erythromycin, bacitracin, neomycin, polymycin B, mupirocin, clindamycin and mixtures thereof. Suitable antiseptics include silver sulfadiazine, chlorhexidine, povidone iodine, triclosan, other silver salts and colloidal silver, sucralfate, quaternary ammonium salts and mixtures thereof.

An especially suitable antimicrobial structure for use as or in the screen structure is a silver impregnated charcoal cloth, for example of the kind described in US-A-4529623. The activated charcoal reduces bioburden in the wound, by trapping bacteria and binding endotoxins which can be detrimental to the wound healing process. Furthermore, the silver impregnated onto the charcoal has bactericidal effect, and the activated charcoal has a primary odour-absorbency function.

In certain embodiments, the screen structure comprises more than one layer of material selected from: active biopolymer layers, hydrogel layers, antimicrobial layers, and support layers.

For example, the screen structure may comprise at least one active layer adapted to remove or inactivate undesirable components from the wound environment and/or to concentrate desirable components present in the wound environment, and at least one support body for the active layer. The support body may, for example, comprise a layer of textile, mesh, foam or gauze. Optionally textile fibers from the support body may extend into the active layer(s). The textile layer may be woven or nonwoven , and can be folded or multiple to provide adequate packing. Such layers provide structural integrity to the active layer.

In certain embodiments the screen structure further comprises a dressing change indicator. This is a region adjacent to the cover sheet, and visible through a transparent region of the cover sheet, that changes appearance when a dressing change is needed. For example, it may change colour when the hydrogel layer of the dressing is saturated with wound fluid.

As already noted, the active layers of the wound dressing according to the present invention may be associated with one or more layers of conventional screen means, for example as described in EP-A-0620720, EP-A-0688189, WO01/89431, and WO2004/037334.

In certain embodiments, the screen structures in the wound dressings according to the present invention comprise or consist of materials that selectively remove components from wound fluid by filtration, affinity binding, or size exclusion.

These materials are typically non-resorbable materials that selectively bind either bad factors such as proteases, or good factors such as growth factors. (The good factors can be released from the binding materials e.g. by buffers or salt and cycled back into the wound either during vacuum treatmemt or after the dressing is removed).

The use of molecular sieve materials to remove water selectively from the wound fluid has been discussed above.

In certain embodiments, the screen selectively binds proteases, in particular matrix metalloproteinases, allowing passage of exudate containing more good factors which is then expected be more mitogenic in cell proliferation tests. For example, Benzamidine Sepharose 6B, (available from Amersham Bioscience) could be included in the screen for this purpose.

At least a portion of the screen releasably binds growth factors but not proteases to the same extent. It has been shown that ORC binds growth factors found in wound fluid. The bound growth factors can then be released back into the wound either by gradual breakdown of the material in vivo (e.g. for ORC), or by the addition of a suitable affinity release serum. HiTrap Heparin HP (available from Amersham Bioscience) could be included in the screen for this purpose.

The screen may comprise an immobilized affinity binding partner (e.g. an antibody) that selectively binds to matrix metalloproteinases and/or other undesirable components of wound fluid. All MMP's have significant homology and a single affinity site may be used to remove all MMP's. The binding partner may be conjugated to a solid substrate, for example by avidin-biotin linkage. A suitable affinity binding partner for matrix metalloproteinases is TIMP-1.

A further embodiment of this invention uses size exclusion chromatography materials in the screen structure, for example reticulated polystyrene beads such as those described in EP-A-088783, for removal of proteases from wound fluid. The screen structure could also include an ultrafiltration (or dialysis) membrane having a molecular weight cut-off adapted to block the passage of proteases but to allow the passage of water and relatively low molecular weight desirable factors such as growth factors. In other embodiments, the ultrafiltration membrane can be used to separate an absorbent or superabsorbent material in the screen structure from the wound fluid. A molecular weight cut-off in the range of from about 10,000 to about 50,000 is suitable for the ultrafiltration/dialysis membrane.

In yet other embodiments the selective materials in the screen structure could comprise ion exchange separation materials (for example DEAE cellulose) to selectively bind ionically charged components (e.g. bacterial endotoxins, growth factors) which could then optionally be returned to the wound following exudate removal by suitable salt treatment of the materials. Suitable ion exchange materials could also be used to buffer the wound to the optimal slightly acidic (pH 4.5 to about 6.5) for wound healing.

In yet other embodiments the selective materials in the screen structure could comprise one or more molecularly imprinted polymers for selective binding of one or more wound fluid components. These polymers have complex surfaces with three-dimensional topologies possessing a specific spatial arrangement of chemical functional groups for selective binding of predetermined molecules. Most of these polymers are created by interaction of a template molecule with either a pre-formed polymer or by polymerization of monomers in the presence of a template. The resulting structures are bulk polymers in which the template leaves behind cavities, which are more or less complementary to the shape and spatial distribution of functional groups of the template.

In certain embodiments of the wound dressing according to the present invention, the screen structure comprises a plurality of relatively movable screen particles enclosed by a structure of sheet material. The particles are typically small beads, for example having a diameter of from about 0.1mm to about 5mm, typically about 0.5mm to about 3mm. The enclosing structure of sheet material is usually liquid permeable, for example an envelope, and the sheet material is typically a perforated thermoplastic sheet or a textile material.

The particles are loosely packed in the envelope so that they are able to move about and conform to the shape of the wound while allowing vacuum to be applied to the wound and functioning as an efficient screen to tissue overgrowth. The scrim prevents the microbeads from being sucked from the wound by the vacuum. The microbeads can move about the wound bed to micro-massage the wound. This could potentially encourage microcirculation stimulation, increasing oxygen and nutrient delivery to cells, resulting in faster wound closure.

Microbeads could be made from either bioabsorbable or non- bioabsorbable materials used either individually or in combination. If made of a non- bioabsorbable material the microbeads could be strung together for ease of removal. They could also be coated with agents that would improve healing.

Using microbeads rather than a sponge may allow the vacuum to be applied more efficiently or evenly over the wound bed. Using microbeads rather than a sponge may allow more efficient extract of the wound fluid due to decreased friction. Microbeads would have a smoother surface than that of a sponge. The use of microbeads as a screen would allow the screen to fit any size or shape of wound cavity. This would remove the need for cutting to size prior to application making the product easier to use and reduce the chance of bacterial contamination.

It is also envisaged that the screen structure according to this aspect of the invention could comprise or consist essentially of microbeads, without a retaining scrim. The microbeads would be easily removed from the wound bed by irrigation if made of a non- bioabsorbable material. This contrasts with sponge screens, which can be difficult to remove due to tissue re-growth into the sponge causing pain to the patient. Tissue re-growth around the microbeads could occur to some extent without effecting removal. Alternatively the beads could be made of a bioabsorbable material negating the need for removal.

The beads may be any of the separation beads described above in relation to affinity separation, ion exchange, or size-exclusion separation. Alternatively or additionally, the beads may be manufactured from, encapsulated in, or coated with any of the active materials discussed above, including any of the biopolymers having selective activity against protease enzymes. Mixtures of different types of beads are also contemplated.

In yet other embodiments, the beads used in the screen structure comprise beads substantially as described in EP-A-0888140, wherein each bead comprises a porous core of a first bioabsorbable material and a substantially non-porous layer of a second bioabsorbable material around the core. The porous core is preferably a sponge formed by freeze-drying a liquid suspension of the first bioabsorbable material. The preferred diameter of the beads is 0.1-4.0 mm, and the beads are preferably dispensed in a liquid or solid matrix.

In other embodiments of the present invention, the screen structure incorporates a separation device comprising: an inlet for wound fluid; a separation member; and an outlet for returning wound fluid to the wound after it has passed through the separation member. That is to say, a device for purification and optional concentration of the wound fluid is provided integrally with the dressing, usually below the cover sheet, and preferably forming part of a larger screen structure. For example, the device may be embedded in a resilient screen layer, for example a layer of hydrophilic foam and/or textile material.

The device normally comprises a housing, for example formed from thermoplastic sheet material. The device comprises an inlet to collect exudate from a wound - this exudate is then treated in the separation member by removing harmful or wound healing inhibiting substances from the exudate. The inlet may, for example, be an opening or a plurality of openings in the housing, normally in the side of the housing that faces the wound under treatment in use. The separation member is usually fitted in the housing for the device. may comprise a bed of separation beads as hereinbefore described, e.g. affinity separation beads, size exclusion separation beads, ion exchange beads, or biopolymer-containing beads. Alternatively or additionally, the separation member may comprise an ultrafiltration or dialysis membrane, for example having a molecular weight cut-off selected around 10,000 to 50,000 so as to remote proteases but allow the passage of growth factors. The device may also comprise a reservoir for storage of the wound fluid inside the housing before, during, or after the purification and concentration steps taking place in the device.

Preferably the device is also adapted to concentrate beneficial substances from the exudate. Accordingly, the device may comprise an absorbent body to absorb water from the wound fluid, and the absorbent body may be covered by a suitable ultrafiltration membrane to prevent absorption of the desirable growth factors.

The housing usually comprises a vacuum port for connection to a source of suction located outside the dressing. The vacuum port communicates with the inlet and optionally with the separation member to draw wound fluid into the device, and preferably to draw the wound fluid through the separation member and optionally into the reservoir. The device may comprise a one-way valve to prevent wound fluid flowing out of the reservoir back to the separation member and the inlet. In certain embodiments, a second vacuum port is provided in the device in communication with the optional absorbent body to draw wound fluid into the absorbent body, for example to draw water from the wound fluid through a size exclusion membrane into the absorbent layer , thereby concentrating the wound fluid. In certain embodiments, the size exclusion membrane is provided between the reservoir as hereinbefore described and the absorbent layer, whereby the vacuum can be switched from the reservoir to the absorbent layer to concentrate the purified wound fluid in the reservoir.

The device may further comprise a pressure inlet (or the vacuum inlet may be used for this purpose) for applying hydraulic or preferably gas pressure to expel purified and optionally concentrated wound fluid from the device through the outlet to the wound. Suitably, the pressure inlet is configured to apply pressure to the reservoir as hereinbefore described. A filtration member may be provided in the reservoir or the outlet conduit to remove solid particles, including cells, from the purified and optionally concentrated wound fluid before it is returned to the wound.

The outlet may comprise a one-way valve to block return of wound fluid to the device through the outlet when vacuum is reapplied to the device to draw wound fluid into the device through the inlet.

In certain embodiments, the wound fluid purification device comprises: a housing; a wound fluid inlet in the housing communicating with the wound facing side of the dressing, through which wound fluid can be drawn into the housing; a vacuum inlet communicating with the outside of the wound dressing for applying suction to an interior region of the housing a bed of a selectively adsorbent material through which the wound fluid can be drawn under suction from the vacuum inlet; a reservoir into which the wound fluid can be drawn under suction from the vacuum inlet; and a wound fluid outlet communicating with the reservoir and with the wound facing side of the dressing, through which purified wound fluid from the reservoir can be returned to the wound from the reservoir. Preferably, the device further comprises an absorbent body to concentrate the wound fluid by selectively removing water from the wound fluid. The absorbent body may, for example, comprise a suitable molecular sieve material. In other embodiments the absorbent body may be separated from the wound fluid by a size exclusion membrane. In these embodiments, the device may further comprise a second vacuum inlet for applying suction to the absorbent body to draw water into the absorbent body through the membrane. The device preferably further comprises a filter to remove particles and cells from the wound fluid, and the filter is preferably located in the reservoir or in the wound fluid outlet.

In another aspect, the present invention provides a kit for assembly into a wound dressing according to the present invention, said kit comprising: a cover configured for placement over the wound to provide a sealed environment around the wound and adapted for communication with a source of vacuum, and a screen structure for placement between the cover and the wound, characterized in that said screen structure comprises a freeze-dried sponge made by freeze-drying or solvent drying a dispersion of oxidized regenerated cellulose (ORC) with collagen or chitosan in water, whereby the screen structure is adapted to remove or inactivate undesirable components from the wound environment and/or to concentrate desirable components present in the wound environment.

The features of the cover and the screen structure may suitably be any of the features as hereinbefore described in relation to the first aspect of the invention. The cover and the screen structure may be packaged together, or separately. One or both of the cover and the screen structure may be sterile and packaged in a microorganism-impermeable container. The use of a kit allows assembly of the wound dressing by the care giver, A given cover may be combined with a screen structure having the most appropriate therapeutic effect at the time of treatment in order to optimize wound healing. It will be appreciated that the kit according to the present invention may comprise a cover and a plurality of loose particles, such as the selectively absorbent beads described hereinbefore. It will also be appreciated that the kit according to the present invention may further comprise a conventional screen material, for example a hydrophilic foam, for use in conjunction with any of the special screen materials described hereinbefore.

In another aspect, the present invention provides a wound treatment system comprising a wound dressing according to the present invention as hereinbefore defined, and a vacuum source for providing said vacuum treatment to a wound.

The wound dressing, kit and system of the present invention may be used for promoting wound healing in a mammal comprising the steps of : applying a wound dressing according to the present invention over a wound in substantially airtight fashion to define a wound treatment space between the cover and a surface of the would, connecting the cover to a vacuum source, and creating a vacuum within the wound treatment space.

The term "vacuum" here and elsewhere in the present specification refers to any pressure below ambient atmospheric pressure. Suitably, the step of applying a vacuum includes lowering the pressure in the wound treatment space to an absolute value of from about 0.1bar to about 0.95bar, suitably from about 0.5 bar to about 0.9 bar and typically to an absolute value of from about 0.75bar to about 0.85bar. The vacuum may be static or dynamic. The vacuum may be applied continuously or intermittently to the wound treatment space, substantially as hereinbefore described.

It will be appreciated that any feature or embodiment of the present invention that is described in relation to any one embodiment is equally applicable to any other embodiment of the invention mutatis mutandis.

Specific embodiments of the present invention will now be discussed further, by way of example, with reference to the accompany drawings, in which:
Figure 1 shows a longitudinal cross-section through a wound dressing according to the first embodiment of the present invention;
Figure 2 shows a longitudinal cross-section through a wound dressing according to a second embodiment of the present invention;
Figure 3 shows a longitudinal cross-section through a wound dressing according to a third embodiment of the present invention;
Figure 4 shows a longitudinal cross-section through a wound dressing according to a fourth embodiment of the present invention;
Figure 5 shows a longitudinal cross-section through a wound dressing according to a fifth embodiment of the present invention;
Figure 6 shows a longitudinal cross-section through a wound dressing according to a sixth embodiment of the present invention; and
Figure 7 shows a detailed longitudinal cross-section through the wound fluid purification device in the embodiment of Figure 6.

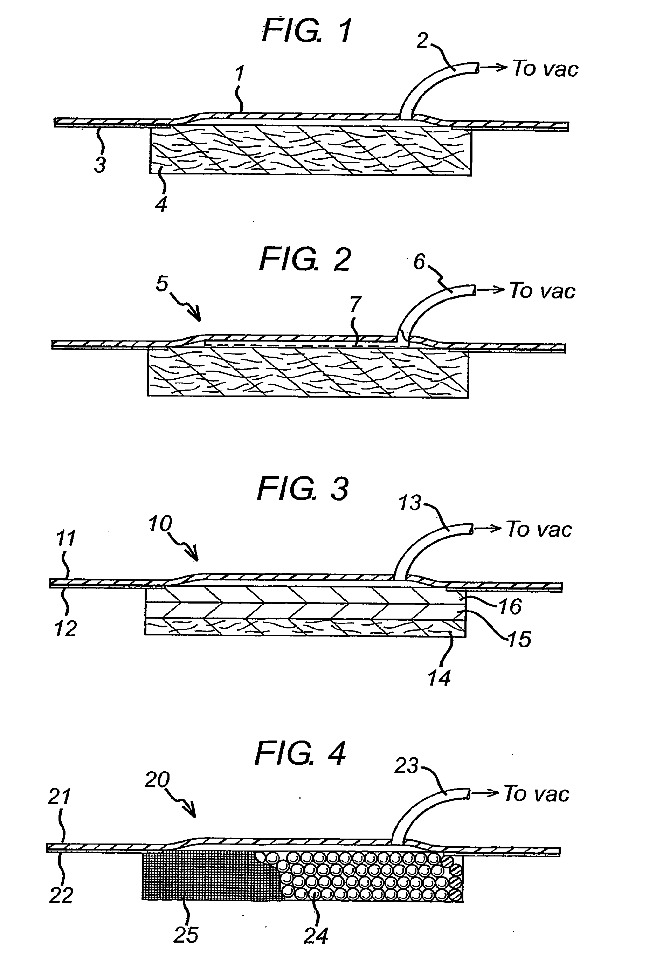

Referring to Figure 1, the wound dressing according to the first embodiment comprises a cover sheet 1 formed of substantially impermeable, thermoformed thermoplastic. A tube 2 passes through the cover sheet 1, for connection to a source of vacuum. A layer of medically acceptable pressure-sensitive adhesive 3 extends around the periphery of the underside of the cover sheet 1, for attachment of the cover sheet to the skin around the wound being treated. The wound dressing further comprises a screen 4, which in this particular embodiment comprises a freeze-dried pad formed from a mixture of fibrillar collagen and oxidised regenerated cellulose (ORC) in the ratio approximately 55:45 by weight, and made substantially as described in EP-A-1153622. These freeze-dried sponges are very light and conformable. The pad 4 is secured to the cover sheet 1 by the adhesive 3. The marginal regions of the adhesive 3 in this and the other embodiments may be protected by a release-coated cover sheet (not shown) prior to use. The dressing is usually packaged in a microorganism impermeable container, and is sterilised e.g. by gamma-irradiation.

It will be appreciated that the embodiment of Figure 1 could also be made up from a kit according to the present invention. The kit would comprise the screen 4 and, separately, the cover sheet, tube and adhesive layer (with cover sheet), packaged separately or together, for assembly at the time of use. The other embodiments shown in Figures 2 to 6 could likewise be made up from a kit in accordance with the present invention.

It will also be appreciated that the freeze-dried sponge screen 4 could be replaced by freeze-dried sponges made from other materials that inactivate matrix metalloproteinases and/or retain and release growth factors. For example, a freeze-dried pad of collagen with alginate or chitosan with ORC. The screen 4 could also be replaced by a liquid-permeable envelope, for example a liquid-permeable non-woven scrim envelope, containing one or more of the active polymers in particulate or fibrillar form.

Referring to Figure 2, the construction of the dressing 5 according to the second embodiment of the invention is generally similar that of the embodiment of Figure 1. However, the embodiment of Figure 2 further includes an air outlet manifold 7 for distributing the suction from the vacuum line 6 across the upper surface of the screen structure. The manifold 7 may, for example, comprise a spirally wound, perforated tube, or any of other suitable manifold structures, for example as described in WO2004/37334. It will be appreciated that the manifold may be positioned on the lower (wound-facing) surface of the screen, or within the screen structure at any point intermediate between the upper and lower surfaces. The use of a manifold enables more uniform suction to be applied to the wound, and draws wound fluid more uniformly into the screen structure.

Referring to Figure 3, the overall structure of the wound dressing 10 according to this embodiment is similar to that of Figure 1, including a cover sheet 11, a vacuum tube 13 and an adhesive layer 12. However, the screen structure in Figure 3 is a laminate made up of three layers 14, 15, 16. Layer 14 is a freeze-dried collagen/ORC sponge as described in relation to Figure 1. Layer 15 is a silver-impregnated charcoal cloth of the kind described for example in US-A-4529623. The activated charcoal is effective to remove bacterial toxins from the wound fluid. Furthermore, the silver impregnated onto the charcoal has bactericidal effect, and the activated charcoal has a primary odour-absorbency function. Layer 16 is a water-absorbent layer. Suitably it is a non-woven fibrous web of hydrophilic textile fibers, e.g. viscose fibers, and superabsorbent polyacrylate gel-forming fibers. Suitably, there is a size exclusion membrane (not shown) separating layers 15 and 16 and having a molecular weight cut-off such that it allows water to pass through into layer 16, but does not allow the passage of growth factors, whereby the growth factors are concentrated in the wound fluid in contact with the wound.

Referring to Figure 4, this embodiment of the wound dressing has similar overall construction to Figure 1 including a cover sheet 21, a vacuum tube 23 and an adhesive layer 22. However, the screen structure of Figure 4 comprises an envelope of a water-permeable, non-woven nylon scrim 25 enclosing a quantity of selectively absorbent beads 24. The selectively absorbent beads typically have a diameter of 1 or 2 mm. They may be chromatography beads, for example size exclusion chromatography beads, ion exchange chromatography beads or affinity chromatography beads specifically adapted to remove or retain predetermined components of the wound fluid. In this embodiment, the beads comprise HiTrap Heparin HP (available from Amersham Bioscience) to selectively and reversibly absorb growth factors. In use, the beads are periodically treated by simple addition of salt and/or buffer of suitable pH to release growth factors back to the wound.

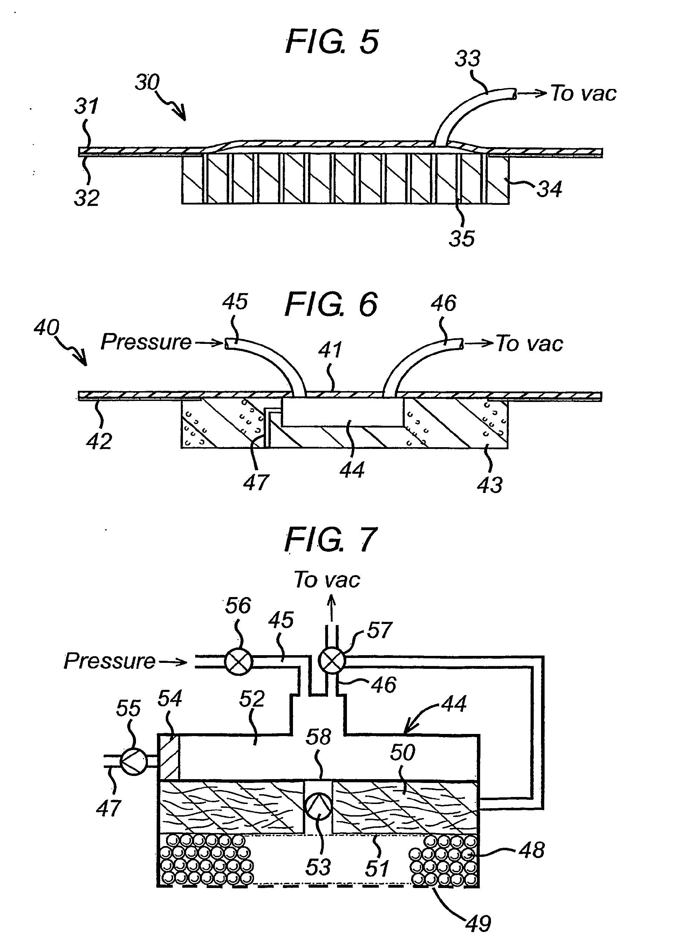

Referring to Figure 5, in this embodiment the dressing 30 comprises a cover sheet 31, and an adhesive layer 32 and a vacuum tube 33 substantially as hereinbefore described in relation to Figure 1. However, the screen structure in the embodiment of Figure 5 comprises a slab of polyacrylate hydrogel 34 having a manifold of air passages 35 formed therein for maintaining vacuum at the wound surface, and to ensure uniform swelling of the hydrogel by the wound fluid.

Referring to Figure 6, the dressing 40 according to this embodiment comprises a semi-rigid, impermeable cover sheet 41 having an adhesive periphery 42 substantially as described in relation to the other embodiments. The screen assembly comprises a foam pad 43. In the embodiment shown in the drawing this pad is formed of an open-cell foam, for example a polyester foam. However, any kind of liquid-permeable pad, for example any of the screen assemblies shown in the embodiments of Figures 1 to 5 would be suitable. The screen assembly of Figure 6 further comprises a wound fluid purification device 44 located inside the wound dressing. The wound fluid purification device 44 is linked to a source of vacuum through vacuum line 46, and to a source of pressurised gas through pressure line 45. A return channel 47 is provided for returning purified and concentrated wound fluid from the device 44 to the wound surface.

Referring to Figure 7, the wound fluid purification device 44 comprises a housing having an apertured base 49, which functions as the inlet through which wound fluid is drawn from the pad into the device. The wound fluid passes through a bed 48 of ion-exchange resin beads for removal of matrix metalloproteinases. The wound fluid then passes through one-way valve 53 into reservoir 52. When the reservoir is full, the vacuum is switched to absorbent layer 50, and water from the reservoir 52 is drawn through size exclusion membrane 58 into absorbent layer 50 to concentrate the higher molecular weight wound healing and growth factors in the wound fluid in reservoir 52. When this process is complete, the concentrated purified wound fluid is returned to the wound through line 47 and valve 55 by applying pressure to the reservoir 52 through pressure line 45. The returning wound fluid passes through the bacterial filter 54 before it is returned to the wound surface.

The above embodiments have been described for purpose of illustration. Many other embodiments falling within the scope of the accompanying claims will be apparent to the skilled reader.

## Claims

1. A wound dressing for vacuum therapy comprising: a cover (1) configured for placement over a wound to maintain a reduced pressure over the wound and adapted for communication with a source of vacuum, and a screen structure (4) for placement between the cover (1) and the wound, **characterized in that** said screen structure (4) comprises a freeze-dried sponge made by freeze-dryiwg or solvent drying a dispersion of oxidized regenerated cellulose (ORC) with collagen or chitosan in water, whereby the screen structure is adapted to remove or inactivate undesirable components from the wound environment and/or to concentrate desirable components present in the wound environment.

2. A wound dressing according to claim 1, wherein the cover (1) is semi-rigid.

3. A wound dressing according to any preceding claim, further comprising tubing (2) for connecting the cover (1) to the vacuum source.

4. A wound dressing according to any preceding claim, wherein said a freeze-dried sponge is made by freeze-drying a dispersion of oxidized regenerated cellulose (ORC) with collagen in water,

5. A wound dressing according to any preceding claim, wherein the screen structure (4) consists of said freeze-dried sponge.

6. A kit for assembly into a wound dressing according to any of claims 1 to 5, said kit comprising: a cover configured for placement over the wound to provide a sealed environment around the wound and adapted for communication with a source of vacuum, and a screen structure for placement between the cover and the wound, **characterized in that** said screen structure comprises a freeze-dried sponge made by freeze-drying or solvent drying a dispersion of oxidized regenerated cellulose (ORC) with collagen or chitosan in water, whereby the screen structure is adapted to remove or inactivate undesirable components from the wound environment and/or to concentrate desirable components present in the wound environment.

7. A wound treatment system comprising a wound dressing according to claim 1 and a vacuum source for providing said vacuum treatment to a wound.

8. The system of claim 7, wherein the vacuum source is programmed to supply vacuum intermittently to the wound treatment space.

9. The system of claim 7, wherein the step of applying a vacuum includes lowering the pressure in the wound treatment space to an absolute value of from 10kpa (0. 1bar) to 90kPa (0.9bar).

10. The system of claim 9, wherein the step of applying a vacuum includes lowering the pressure in the wound treatment space to an absolute value of from 75kPa (0.75bar) to 85kPa (0.85bar).

## Patentansprüche

1. Wundverband für die Vakuumtherapie, der Folgendes umfasst: eine Abdeckung (1) zur Platzierung über einer Wunde zur Aufrechterhaltung eines Unterdrucks über der Wunde und zur Verbindung mit einer Vakuumquelle, und eine Siebstruktur (4) zur Platzierung zwischen der Abdeckung (1) und der Wunde, **dadurch gekennzeichnet, dass** die Siebstruktur (4) einen gefriergetrockneten Schwamm umfasst, der durch Gefriertrocknung oder Lösungsmitteltrocknung einer Dispersion von oxidierter regenerierter Cellulose (ORC) mit Kollagen oder Chitosan in Wasser hergestellt wurde, wodurch die Siebstruktur unerwünschte Bestandteile aus dem Wundmilieu entfernen oder inaktivieren kann und/oder erwünschte Bestandteile, die im Wundmilieu vorliegen, konzentrieren kann.

2. Wundverband nach Anspruch 1, worin die Abdeckung (1) halbstarr ist.

3. Wundverband nach einem der vorhergehenden Ansprüche, der ferner einen Schlauch (2) zur Verbindung der Abdeckung (1) mit der Vakuumquelle umfasst.

4. Wundverband nach einem der vorhergehenden Ansprüche, worin der gefriergetrocknete Schwamm durch Gefriertrocknung einer Dispersion von oxidierter regenerierter Cellulose (ORC) mit Kollagen in Wasser hergestellt wird.

5. Wundverband nach einem der vorhergehenden Ansprüche, worin die Siebstruktur (4) aus dem gefriergetrockneten Schwamm besteht.

6. Set zur Anordnung in einem Wundverband nach einem der Ansprüche 1 bis 5, worin das Set Folgendes umfasst: eine Abdeckung zur Platzierung über der Wunde zur Bereitstellung eines abgedichteten Milieus um die Wunde und zur Kommunikation mit einer Vakuumquelle, und einer Siebstruktur zur Platzierung zwischen der Abdeckung und der Wunde, **dadurch gekennzeichnet, dass** die Siebstruktur einen gefriergetrockneten Schwamm umfasst, der durch Gefriertrocknung oder Lösungsmitteltrocknung einer Dispersion von oxidierter regenerierter Cellulose (ORC) mit Kollagen oder Chitosan in Wasser hergestellt wurde, wodurch die Siebstruktur unerwünschte Bestandteile aus dem Wundmilieu entfernen oder inaktivieren kann und/oder erwünschte Bestandteile, die im Wundmilieu vorliegen, konzentrieren kann.

7. Wundbehandlungssystem, das einen Wundverband nach Anspruch 1 und eine Vakuumquelle zur Bereitstellung der Vakuumbehandlung für eine Wunde umfasst.

8. System nach Anspruch 7, worin die Vakuumquelle so programmiert ist, dass sie den Wundbehandlungsraum intermittierende mit Vakuum versorgt.

9. System nach Anspruch 7, worin der Schritt der Applikation von Vakuum die Absenkung des Drucks im Wundbehandlungsraum auf einen absoluten Wert von 10 kPa (0,1 bar) bis 90 kPa (0,9 bar) einschließt.

10. System nach Anspruch 9, worin der Schritt der Applikation von Vakuum die Absenkung des Drucks im Wundbehandlungsraum auf einen absoluten Wert von 75 kPa (0,75 bar) bis 85 kPa (0,85 bar) einschließt.

## Revendications

1. Pansement pour thérapie en dépression, comprenant :
une couverture (1) configurée pour être placée sur une blessure, permettant de maintenir une dépression sur la blessure et conçue pour communiquer avec une source de dépression et
une structure d'écran (4) destinée à être placée entre la couverture (1) et la blessure,
**caractérisé en ce que**
la structure d'écran (4) comprend une éponge lyophilisée formée en lyophilisant ou en séchant à l'aide d'un solvant une dispersion de cellulose oxydée régénérée ("oxidized regenerated cellulose" - ORC) avec du collagène ou du chitosan dans de l'eau et
**en ce que** la structure d'écran est conçue pour enlever ou inactiver des composants indésirables de l'environnement de la blessure et/ou pour concentrer les composants souhaitables présents dans l'environnement de la blessure.

2. Pansement selon la revendication 1, dans lequel la couverture (1) est semi-rigide.

3. Pansement selon l'une quelconque des revendications précédentes, comprenant de plus un tubage (2) qui raccorde la couverture (1) à la source de dépression.

4. Pansement selon l'une quelconque des revendications précédentes, dans lequel l'éponge lyophilisée est formée par lyophilisation d'une dispersion de cellulose oxydée régénérée ("oxidized regenerated cellulose" - ORC) avec du collagène dans de l'eau.

5. Pansement selon l'une quelconque des revendications précédentes, dans lequel la structure d'écran (4) est constituée de ladite éponge lyophilisée.

6. Trousse d'assemblage d'un pansement selon l'une quelconque des revendications 1 à 5, ladite trousse comprenant :
une couverture configurée pour être placée sur une blessure, permettant de maintenir une dépression sur la blessure et conçue pour communiquer avec une source de dépression et
une structure d'écran destinée à être placée entre la couverture et la blessure,
**caractérisée en ce que**
la structure d'écran comprend une éponge lyophilisée formée en lyophilisant ou en séchant à l'aide d'un solvant une dispersion de cellulose oxydée régénérée ("oxidized regenerated cellulose" - ORC) avec du collagène ou du chitosan dans de l'eau, la structure d'écran étant conçue pour enlever ou inactiver des composants indésirables de l'environnement de la blessure et/ou pour concentrer les composants souhaitables présents dans l'environnement de la blessure.

7. Dispositif de pansement comprenant un pansement selon la revendication 1 et une source de dépression permettant de conduire ledit traitement en dépression d'une blessure.

8. Dispositif selon la revendication 7, dans lequel la source de dépression est programmée pour appliquer une dépression de façon intermittente sur l'espace de traitement de la blessure.

9. Dispositif selon la revendication 7, dans lequel l'étape qui consiste à appliquer la dépression comprend l'action qui consiste à abaisser la pression dans l'espace de traitement de la blessure jusqu'à une valeur absolue comprise entre 10 kPa (0,1 bar) et 90 kPa (0,9 bar).

10. Dispositif selon la revendication 9, dans lequel l'étape qui consiste à appliquer une dépression comprend l'action qui consiste à abaisser la pression dans l'espace de traitement de la blessure jusqu'à une valeur absolue comprise entre 75 kPa (0,75 bar) et 85 kPa (0,85 bar).
